(19) Europäisches Patentamt
European Patent Office
Office européen des brevets

(11) **EP 3 929 549 B1**

(12) **FASCICULE DE BREVET EUROPEEN**

(45) Date de publication et mention
de la délivrance du brevet:
**19.11.2025 Bulletin 2025/47**

(21) Numéro de dépôt: **21180972.8**

(22) Date de dépôt: **22.06.2021**

(51) Classification Internationale des Brevets (IPC):
**G01J 5/00** *(2022.01)* **G01J 5/04** *(2006.01)*
**G01J 5/08** *(2022.01)* **G01J 5/52** *(2022.01)*

(52) Classification Coopérative des Brevets (CPC):
**G01J 5/0025; G01J 5/04; G01J 5/0859; G01J 5/53;**
G01J 2005/0077

(54) **SYSTÈME ET DISPOSITIF DE MESURE SANS CONTACT DE LA TEMPÉRATURE CORPORELLE D'UN INDIVIDU**

KONTAKTLOSES SYSTEM UND KONTAKTLOSE VORRICHTUNG ZUM MESSEN DER KÖRPERTEMPERATUR EINER PERSON

SYSTEM AND DEVICE FOR CONTACTLESS MEASUREMENT OF THE BODY TEMPERATURE OF AN INDIVIDUAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.06.2020 FR 2006484**

(43) Date de publication de la demande:
**29.12.2021 Bulletin 2021/52**

(73) Titulaires:
• **Manneschi, Alessandro**
**52100 Arezzo (IT)**
• **Manneschi, Luca**
**52100 Arezzo (IT)**

(72) Inventeurs:
• **Manneschi, Alessandro**
**52100 Arezzo (IT)**
• **Manneschi, Luca**
**52100 Arezzo (IT)**

(74) Mandataire: **Regimbeau**
**20, rue de Chazelles**
**75847 Paris Cedex 17 (FR)**

(56) Documents cités:
**EP-A1- 2 577 242        EP-A1- 3 633 335**
**EP-B1- 2 871 452        WO-A1-2018/194658**
**WO-A2-2004/097389        US-A1- 2007 153 871**
**US-B1- 6 447 160**

• **HGH INFRARED SYSTEMS - EOI: "Accurate Fever Detection and Temperature Measurement with the new CN-37 Blackbody", YOUTUBE, 22 May 2020 (2020-05-22), pages 1 pp., XP054981515, Retrieved from the Internet <URL:https://www.youtube.com/watch?v=tkFK5TeNz50> [retrieved on 20210310]**

## Description

### DOMAINE DE L'INVENTION

L'invention concerne de manière générale la mesure de la température corporelle d'un individu, notamment à l'entrée d'une zone à accès limité tel qu'un bâtiment public ou privé.

### ETAT DE LA TECHNIQUE

Le contexte sanitaire actuel a révélé la nécessité de pouvoir contrôler rapidement la température corporelle d'individus souhaitant accéder à une zone particulière, telle que l'entrée d'un bâtiment public ou privé. En particulier, il est souhaitable de pouvoir identifier les personnes dont la température corporelle est supérieure à 37,5°C afin de limiter les risques de propagation de virus (tels que le coronavirus COVID-19).

Dans le domaine médical, on connaît des thermomètres portatifs de contrôle de la température par contact, qui présentent l'avantage d'être économiques et précis. Toutefois, ces thermomètres nécessitent un contact physique avec la peau de l'individu à contrôler, ce qui implique un temps de mesure relativement élevée et la nécessité de remplacer, à chaque mesure, une capsule d'isolation du thermomètre afin d'assurer l'hygiène du test.

Il existe également des systèmes de contrôle infrarouge portatif du type « à pistolet ». Ces systèmes permettent à un opérateur de mesurer sans contact la température d'un individu en étant placé à proximité de l'individu à contrôler. La température est généralement mesurée au niveau du front ou sur les poignets de l'individu, de façon très rapide. Toutefois, le système étant sous la forme d'un pistolet, il doit être manipulé par un opérateur dédié. De plus, la précision du capteur infrarouge est variable dans le temps en raison des variations thermiques environnementales et de la dérive des caractéristiques du capteur infrarouge. Pour améliorer la précision du système, il serait nécessaire de le calibrer périodiquement, ce qui est lourd pour un système portatif de ce type. De plus, la distribution de la température sur le visage est variable en fonction de son exposition à l'environnement externe (par exemple, vent ou soleil) et selon les individus. Or, un opérateur ne peut pas déterminer a priori la zone du visage ayant la température maximale, de sorte que la température mesurée ne correspond pas nécessairement à la température corporelle réelle de l'individu.

Il existe également des systèmes de contrôle infrarouge à installation fixe, comprenant une caméra infrarouge montée sur un support, dont l'axe optique est parallèle au sol et au niveau de la hauteur moyenne d'un visage. Toutefois, il ressort que ces systèmes souffrent des mêmes lacunes que les systèmes portatifs même si, étant fixes, leur fonctionnement peut être plus complexe pour en améliorer la précision de mesure. En particulier, le comportement des capteurs infrarouges de ces systèmes fixes est stabilisé par un contrôle thermique de la température mesurée par le capteur infrarouge et par l'utilisation périodique et parfois permanente d'un corps noir externe utilisé comme référence dont l'émissivité est unitaire et la température connue. Néanmoins, il ressort que l'utilisation d'un corps noir externe pose souvent des problèmes d'installation qui peuvent rendre complexe l'installation du poste de contrôle du système. Par ailleurs, le corps noir peut subir des interférences (passage d'individus par exemple). De plus, il est nécessaire qu'un opérateur gère manuellement le moment où l'individu dont on veut déterminer la température corporelle se trouve à une distance appropriée pour effectuer la mesure et qu'il synchronise la lecture de la température avec un instant de passage de l'individu. Enfin, lorsque les mesures sont effectuées à l'entrée d'une zone à accès limitée, il apparaît difficile d'isoler l'individu pour lequel la mesure de température est réalisée et d'ignorer les individus passant à côté, alors que ces individus forment eux aussi une source de chaleur et sont donc susceptibles de perturber les mesures.

Les documents US 2007/153871 A1 et WO 2004/097389 A2 divulguent des exemples de l'état de la technique.

### EXPOSE DE L'INVENTION

Un but de l'invention est de remédier aux inconvénients précités.

En particulier, un but de l'invention est de proposer un système de mesure sans contact de la température corporelle d'un individu qui soit fiable, stable dans le temps et fournisse une valeur précise de la température corporelle de l'individu, quel que soit l'environnement de mesure.

Un autre but de l'invention est de proposer un système de mesure de la température corporelle d'un individu qui puisse facilement être transporté par un opérateur et installé rapidement au niveau d'un accès donné et qui ne nécessite pas de manipulation par l'opérateur lors de la mesure de la température.

Un autre but encore de l'invention est de proposer un système de mesure permettant de limiter les perturbations de l'environnement, malgré la présence éventuelle d'autres individus que celui pour lequel on cherche à déterminer la température corporelle.

Il est à cet effet proposé, selon un premier aspect de l'invention un système de mesure d'une température corporelle d'un individu selon la revendication 1.

Certaines caractéristiques préférées mais non limitatives du système de mesure selon le premier aspect sont les suivantes, prises individuellement ou en combinaison :

- le premier étalon, le deuxième étalon, la première sonde thermique et la deuxième sonde thermique sont montées sur le portique ;

- l'unité de traitement est en outre configurée pour appliquer un coefficient de compensation prédéterminé à la valeur de température de chaque pixel image infrarouge ;
- le système de mesure comprend en outre une caméra à spectre visible comprenant une puce de détection de spectre visible comprenant une matrice de pixels visibles et étant configurée pour générer une image visible comprenant une pluralité de pixels images visibles, la caméra à spectre visible étant fixée sur le portique de sorte qu'un champ visuel de la caméra à spectre visible couvre au moins la portion du canal de passage, l'unité de traitement étant en outre configurée pour identifier les pixels image visibles correspondant à au moins une partie d'un visage d'un individu, pour mettre en correspondance l'image visible et l'image électronique de sorte à identifier les pixels images infrarouges correspondant aux pixels images visibles identifiés, pour déterminer la valeur de température maximale associée aux pixels image infrarouges identifiés ;
- la caméra infrarouge et la caméra à spectre visible sont montées sur un bras fixé sur la traverse et s'étendant depuis une sortie du portique ;
- le système de mesure comprend en outre un capteur de présence configuré pour déterminer une présence d'un individu dans le canal de passage ;
- le premier étalon et le deuxième étalon sont fixés sur l'un parmi les deux panneaux latéraux et la traverse ;
- le premier étalon présente une première température de référence et le deuxième étalon présente une deuxième température de référence différente de la première température de référence ;
- la première température de référence et la deuxième température de référence sont comprises entre 35°C et 40°C ;
- le système de mesure comprend en outre un premier élément de chauffe et un deuxième élément de chauffe configurés pour maintenir le premier étalon et le deuxième étalon à la première température de référence et à la deuxième température de référence, respectivement ;
- le système de mesure comprend en outre une unité de signalisation configurée pour générer une alerte lorsque la valeur de température maximale est supérieure à un seuil prédéterminé ;
- l'unité de traitement est configurée pour identifier les pixels image visibles correspondant aux yeux de l'individu, de préférence un coin interne d'au moins un oeil de l'individu ; et/ou
- le système de mesure comprend en outre une lumière, de préférence clignotante, fixée à proximité de la caméra infrarouge de sorte à attirer un regard d'un individu transitant dans le canal de passage.

Selon un deuxième aspect, l'invention propose un procédé de mesure d'une température corporelle d'un individu à l'aide d'un système de mesure selon le premier aspect, selon la revendication 10.

Certaines caractéristiques préférées mais non limitatives du procédé de mesure selon le deuxième aspect sont les suivantes, prises individuellement ou en combinaison :

- le procédé comprend en outre les étapes suivantes :

    S2 : réaliser une image visible de tout ou partie de la portion du canal de passage, l'image visible comprenant une pluralité de pixels images visibles ;
    S3 : identifier les pixels image visibles correspondant à au moins une partie d'un visage de l'individu, de préférence au moins un coin interne des yeux ;
    S4 : mettre en correspondance l'image visible et l'image électronique de sorte à identifier les pixels images infrarouges correspondant aux pixels images visibles identifiés à l'étape S3 ; l'étape S9 étant mise en oeuvre sur les pixels image infrarouges identifiés à l'étape S3 ;

- les étapes S1 et S2 sont simultanées ;
- le procédé comprend en outre l'application d'un coefficient de compensation prédéterminé à la valeur de température de chaque pixel image ou à la valeur de température maximale déterminée à l'étape S9 ;
- le procédé comprend en outre, préalablement à l'étape S1, une étape S0 de détermination d'une présence d'un individu dans le portique, les étapes S1 à S9 n'étant mises en oeuvre que lorsqu'un individu est présent dans le canal de passage ;
- le procédé comprend en outre une étape de génération d'une alerte lorsque la valeur de température maximale est supérieure à un seuil prédéterminé ; et/ou
- au cours de l'étape S3, les pixels image visibles correspondant aux yeux de l'individu sont identifiés, de préférence les pixels image visibles correspondant à au moins un coin interne des yeux de l'individu.

**DESCRIPTION DES FIGURES**

D'autres caractéristiques, buts et avantages de l'invention ressortiront de la description qui suit, qui est purement illustrative et non limitative, et qui doit être lue en regard des dessins annexés sur lesquels :

    La figure 1 illustre de façon schématique un exemple de réalisation d'un système de mesure conforme à un mode de réalisation de l'invention ;

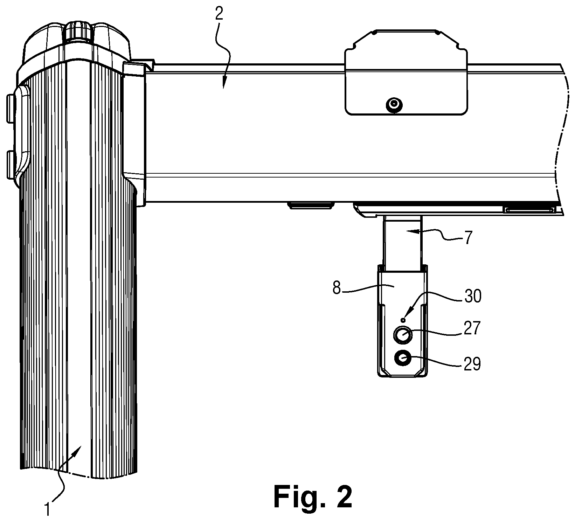

    La figure 2 est une vue de face partielle du système de mesure de la figure 1 ;

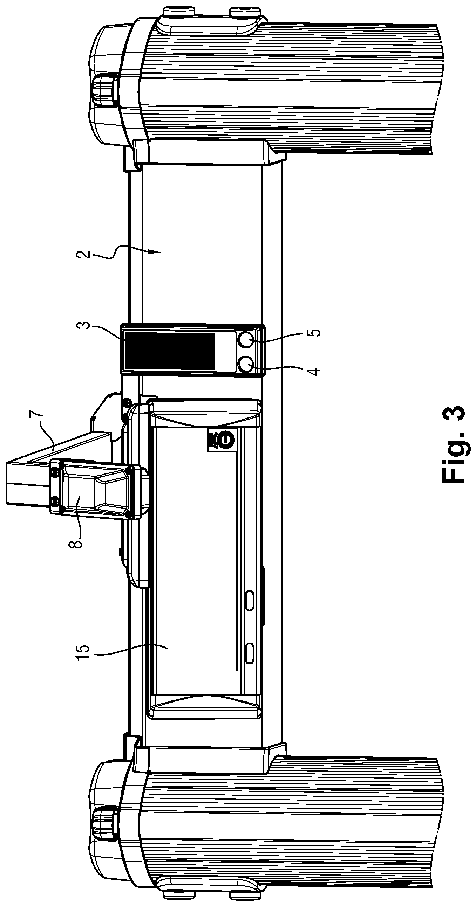

    La figure 3 est une vue arrière partielle du système de mesure de la figure 1 ;

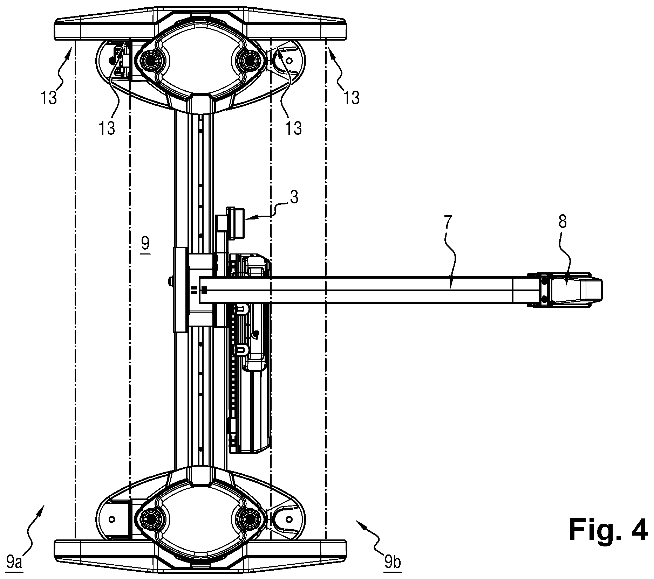

    La figure 4 est une vue du dessus du système de mesure de la figure 1, sur laquelle ont été représentés en traits discontinus les faisceaux lumineux de quatre barrières photoélectriques ;

La figure 5 est une vue de côté du système de mesure de la figure 1, sur laquelle ont été représentés des exemples de champs visuels de la caméra infrarouge et de la caméra à spectre visible ;

La figure 6 est une vue en éclaté d'un exemple de réalisation d'un module de calibration ;

La figure 7 est une vue en éclaté d'un exemple de réalisation d'un boîtier comprenant la caméra infrarouge et la caméra à spectre visible d'un système de mesure conforme à l'invention.

La figure 8 illustre un exemple d'image électronique pouvant être obtenue par la caméra infrarouge du dispositif de mesure de la figure 1, sur laquelle a été illustrée en détail et de manière schématique un exemple de module de calibration ;

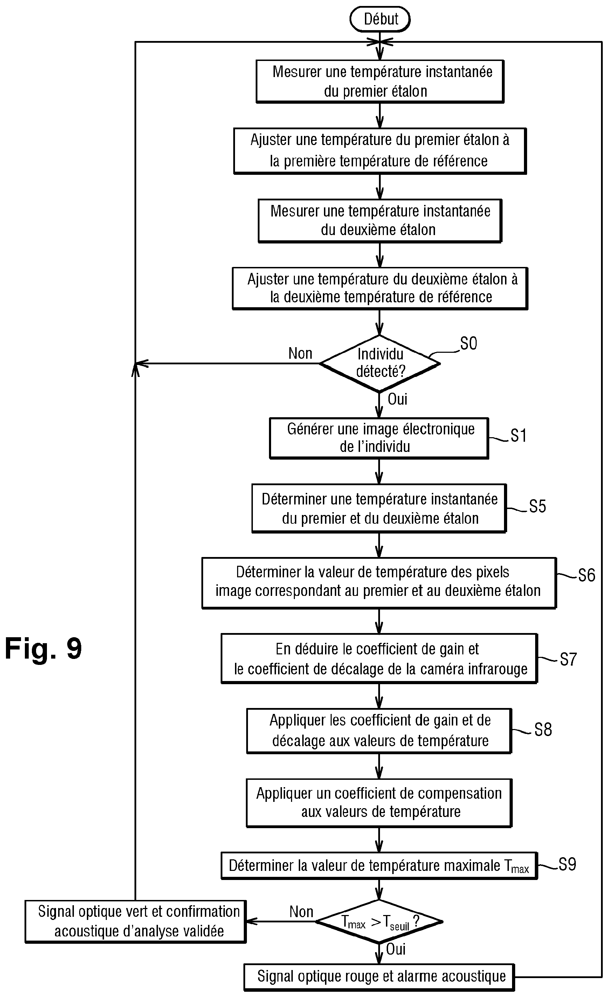

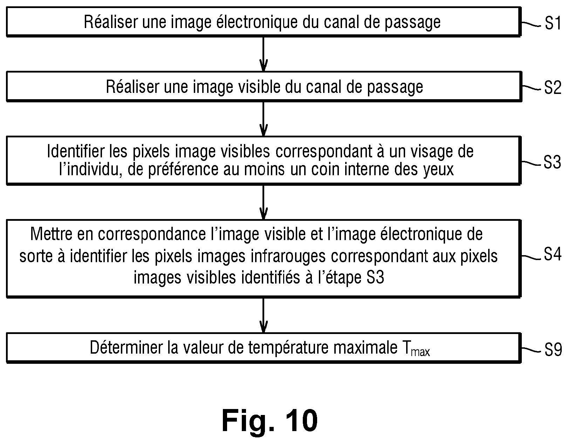

Les figures 9 et 10 sont des organigrammes d'étapes d'un procédé de mesure selon un mode de réalisation de l'invention.

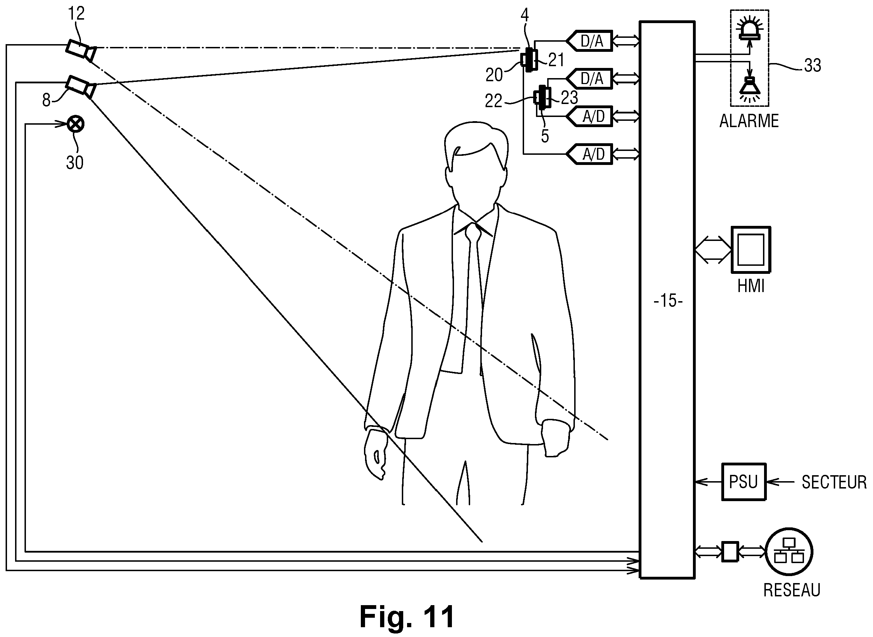

La figure 11 est un schéma synoptique d'un exemple de réalisation d'un système de mesure conforme à l'invention.

Sur l'ensemble des figures, les éléments similaires portent des références identiques.

## DESCRIPTION DETAILLEE DE L'INVENTION

Afin de réaliser des mesures fiables, stables et sans contact de la température d'un individu, l'invention propose un système de mesure 10 d'une température corporelle d'un individu comprenant :

- un portique 11 ;
- une caméra infrarouge 12 ;
- une caméra à spectre visible 6 ;
- optionnellement, un capteur de présence 13 et/ou une lumière 30, de préférence clignotante ; et
- une unité de traitement 15 configurée pour déterminer une température corporelle de l'individu.

Portique 11

Le portique 11 comprend deux panneaux latéraux 1 reliés par une traverse 2 et délimitant ensemble un canal de passage 9 pour un individu. Les panneaux latéraux 1 et la traverse 2 sont raccordés mécaniquement de sorte à être monobloc.

Optionnellement, le portique 11 peut comprendre une traverse 2 supplémentaire, sensiblement parallèle à la traverse 2 et reliant également les deux panneaux latéraux 1.

Chaque panneau latéral 1 présente une face interne orientée vers le canal de passage 9. Plus précisément, la face interne du premier panneau fait face à la face interne du deuxième panneau de sorte à délimiter latéralement le canal. Les panneaux latéraux 1 présentent en outre chacun une première extrémité, ou extrémité d'entrée, qui délimitent ensemble une entrée 9a dans le canal pour un individu, et une deuxième extrémité, ou extrémité de sortie, qui est opposée à l'extrémité d'entrée et définit la sortie 9b du canal 9. Le canal de passage 9 est donc délimité par l'entrée 9a et la sortie 9b du portique 11, l'entrée 9a et la sortie 9b étant définis par le sens de circulation d'un individu dans le portique 11.

Le portique 11 comprend en outre un support, monté sur l'un parmi les panneaux latéraux 1 et la traverse 2 et configuré pour recevoir la caméra infrarouge 12 et la caméra à spectre visible 6. Dans une forme de réalisation, le support comprend un bras 7, comprenant une première extrémité montée sur le portique 11, de préférence au niveau de la sortie 9b du portique 11, et une deuxième extrémité libre, opposée à la première extrémité et s'étendant dans le prolongement du portique 11. La caméra infrarouge 12 et la caméra à spectre visible 6 peuvent par exemple être montée sur la deuxième extrémité du bras 7 de sorte à être orientée vers l'intérieur du canal de passage 9.

Le bras 7 permet ainsi de monter et d'orienter facilement les caméras 6, 12 vers l'intérieur du passage. Par ailleurs, cette configuration permet de déporter les caméras infrarouge 12 et à spectre visible 6 vers l'aval du portique 11 et donc de garantir que les images réalisées comprennent d'images de l'individu, lorsque celui-ci est au sein du passage. Le champ visuel 31, 32 des caméras 6, 12 peut en effet couvrir davantage le canal de passage 9 que si elles étaient montées directement sur la traverse 2 ou les panneaux latéraux 1.

De préférence, le bras 7 est monté sur la traverse 2 au niveau de la sortie 9b du portique 11 et s'étend dans le perpendiculairement à la traverse 2, en aval du canal de passage 9.

La mise en œuvre d'un portique 11 délimitant un canal de passage 9 pour l'individu permet avantageusement de garantir qu'un seul individu à la fois se trouve dans le champ visuel 31, 32 de la caméra infrarouge 12 et de la caméra à spectre visible 6, et d'améliorer ainsi la précision des mesures effectuées.

Caméra infrarouge 12

La caméra infrarouge 12 est configurée pour réaliser une image électronique de l'individu. Elle comprend à cet effet une puce de détection infrarouge 25 comprenant un processeur (ou microprocesseur) et une matrice de pixels (ci-après, des pixels « infrarouges »), et un système optique 26, 27 configuré pour focaliser une énergie infrarouge sur la matrice de pixels infrarouges. Chaque pixel infrarouge de la matrice est configuré pour générer un signal électrique en fonction d'une énergie infrarouge entrant par le système optique lors de la réalisation de l'image électronique. Ce signal électrique est transmis au processeur de la puce de détection infrarouge 25 qui le convertit en une valeur de température correspondante. Le processeur génère une image électronique comprenant une pluralité de pixels images infrarouge, chaque pixel image infrarouge étant représentatif de la valeur de température reçue par un pixel infrarouge correspondant de la matrice. Cette image électronique peut le cas échéant être transmise et affichée sur un écran sous la forme d'une carte en couleurs représentant la température apparente de l'individu.

Les pixels infrarouges de la puce de détection infrarouge 25 sont configurés pour détecter une énergie infrarouge présentant une longueur d'onde supérieure ou égale à huit micromètres et inférieure ou égale à quatorze micromètres. Chaque pixel infrarouge peut présenter une largeur maximale supérieure ou égale à cinq micromètres et inférieure ou égale à cent micromètres, selon la résolution totale de la caméra infrarouge 12 recherchée. Cet intervalle de résolution permet en effet de mesurer avec précision la température de zones de l'individu présentant une faible surface, telles que le coin interne de l'œil.

La puce de détection infrarouge 25 peut être un système micro-électromécanique MEMS (acronyme anglais pour microelectromechanical system).

La puce de détection infrarouge 25 est fixée sur un circuit imprimé 24. Dans une forme de réalisation, le circuit imprimé 24 comprend un plot dans lequel est formé une cavité débouchante. La puce de détection infrarouge 25 est alors montée dans la cavité.

Le système optique comprend une ou plusieurs lentilles 26 positionnées sur l'axe optique de la caméra infrarouge 12 et configurées pour focaliser une énergie infrarouge sur la puce de détection infrarouge 25. Par exemple le système optique peut comprendre une lentille 26, montée dans la cavité devant la puce de détection infrarouge 25. Le système optique comprend en outre un diaphragme 27 positionné sur l'axe optique de la lentille 26 et configuré pour délimiter une zone du passage d'un faisceau infrarouge. Le cas échéant, le diaphragme 27 peut être monté sur ou devant le plot de sorte à fermer la cavité logeant la puce de détection infrarouge 25 et l'isoler de l'environnement extérieur.

La lentille 26 et le diaphragme 27 peuvent par exemple être réalisés en germanium afin de laisser passer les radiations infrarouges.

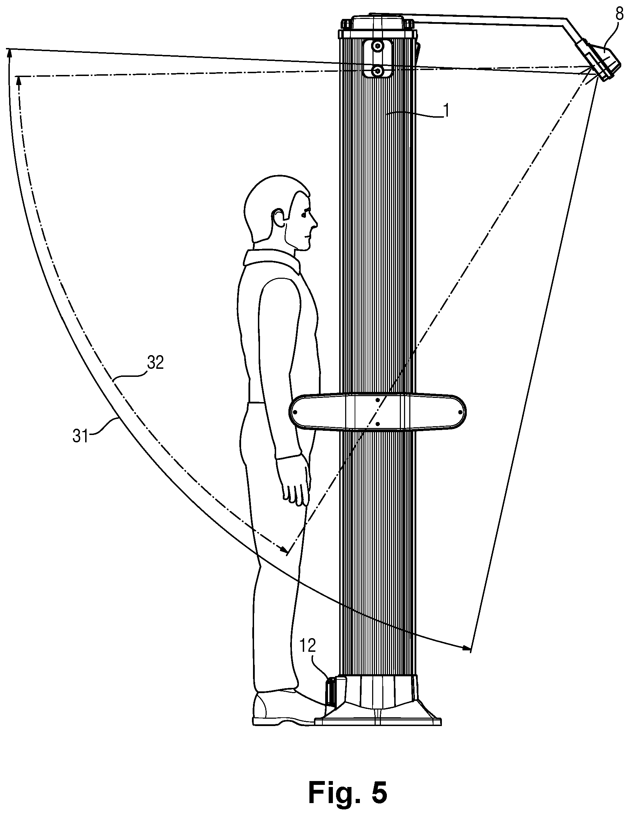

La caméra infrarouge 12 est fixée sur le portique 11 de sorte que son champ visuel 32 couvre au moins une portion du canal de passage 9, de préférence au moins la portion supérieure du canal de passage 9 qui est destinée à comprendre le visage et éventuellement le torse de l'individu (voir par exemple figure 5). Par portion supérieure, ou comprendra ici la portion du canal de passage 9 située du côté de la traverse 2 du portique 11. Le champ visuel 32 de la caméra infrarouge 12 est défini par un angle vertical (φ) et un angle horizontal (θ) (vertical et horizontal étant définis par rapport à l'orientation du portique 11 pendant son fonctionnement, c'est-à-dire lorsque le portique 11 est installé sur le sol ou un support et effectue des mesures de température). Afin d'optimiser la mesure de température, l'angle vertical (φ) et l'angle horizontal (θ) sont supérieurs ou égal 30° et inférieur ou égal à 120° afin de limiter les risques que le champ visuel 32 de la caméra infrarouge 12 couvre l'environnement et de garantir que le visage d'un individu se trouve dans le champ visuel 32 de la caméra infrarouge 12 lorsque celui-ci traverse 2 le canal de passage 9, quelle que soit sa taille.

Caméra à spectre visible 6

La caméra à spectre visible 6 est configurée pour réaliser une image visible de l'individu. Elle comprend à cet effet une puce de détection du spectre visible 29 comprenant une matrice de pixels (ci-après, des pixels « visibles ») et un système optique configuré pour focaliser un rayonnement électromagnétique visible sur la matrice de pixels visibles. Chaque pixel visible de la matrice est configuré pour générer un signal électrique en fonction d'un rayonnement visible entrant par le système optique lors de la réalisation de l'image visible. Ce signal électrique est transmis à un processeur (ou microprocesseur) de la puce de détection du spectre visible 29, qui le convertit en une couleur correspondante. Le processeur génère une image visible comprenant une pluralité de pixels images visibles, chaque pixel image visible étant représentatif du rayonnement visible reçu par un pixel visible correspondant de la matrice.

Les pixels visibles de la puce de détection du spectre visible 29 sont configurés pour détecter un rayonnement visible présentant une longueur d'onde supérieure ou égale à 0,4 micromètres et inférieure ou égale à 0,7 micromètres. Chaque pixel visible peut présenter une largeur maximale supérieure ou égale à un micromètre et inférieure ou égale à trente micromètres, selon la résolution totale de la caméra à spectre visible 6 recherchée. Cet intervalle de résolution permet en effet d'obtenir une image dans laquelle les zones de l'individu présentant une faible surface, telles que le coin interne de l'œil, sont identifiables avec précision.

La puce de détection du spectre visible 29 peut être un système micro-électromécanique MEMS (acronyme anglais pour microelectromechanical system).

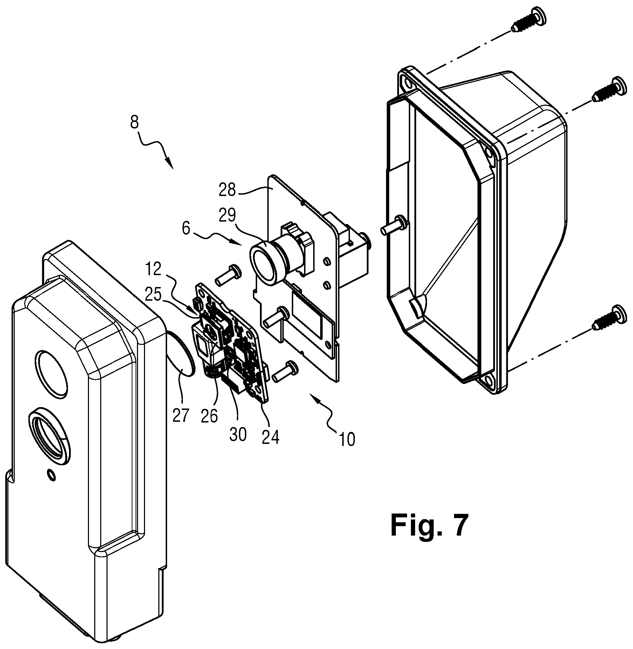

La puce de détection du spectre visible 29 est fixée sur un circuit imprimé 28, comme illustré en figure 7. Le cas échéant, la puce de détection du spectre visible 29 peut être fixée sur le même circuit imprimé 24 que la puce de détection infrarouge 25.

Le système optique de la caméra à spectre visible 6 est conventionnel et comprend, de manière connue en soi, une ou plusieurs lentilles positionnées sur l'axe optique de la caméra à spectre visible 6 et configurées pour focaliser un rayonnement visible sur la puce de détection du spectre visible 29.

La caméra à spectre visible 6 est fixée sur le portique 11 de sorte que son champ visuel 31 couvre au moins la portion du canal de passage 9 qui est couverte par le champ visuel 32 de la caméra infrarouge 12. Le cas échéant, le champ visuel 31 de la caméra à spectre visible 6 peut être plus grand que le champ visuel 32 de la caméra infrarouge 12.

Le ou les circuit(s) imprimé(s) sur lesquels sont fixés les caméras infrarouge 12 et à spectre visible 6 sont logés dans un boîtier 8 comprenant une base, sur laquelle sont fixés le ou les circuits imprimés 28, 24, et un couvercle 23 rapporté et fixé sur la base. Le couvercle 23 comprend une première ouverture, positionnée face à la caméra infrarouge 12 et une deuxième ouverture positionnée face à la caméra à spectre visible 6. En variante, une unique ouverture positionne face aux deux caméras 6, 12 peut être réalisée dans le couvercle 23.

Le diaphragme 27 peut notamment être monté entre la caméra infrarouge 12 et le couvercle 23.

Dans une variante de réalisation, le système de mesure 10 comprend uniquement une caméra infrarouge 12, 6 (ou deux caméras infrarouges 12, 6), la caméra infrarouge 6 implémentant en outre les fonctions déroulées par la caméra à spectre visible 6.

### Unité de traitement 15

L'unité de traitement 15 peut notamment comprendre un calculateur de type processeur, microprocesseur, microcontrôleur, etc., configuré pour exécuter des instructions et contrôler le processeur de la puce de détection infrarouge 25, la caméra à spectre visible 6 et, optionnellement, un capteur de présence 13 et/ou au moins une unité de signalisation 33 (détaillés par la suite).

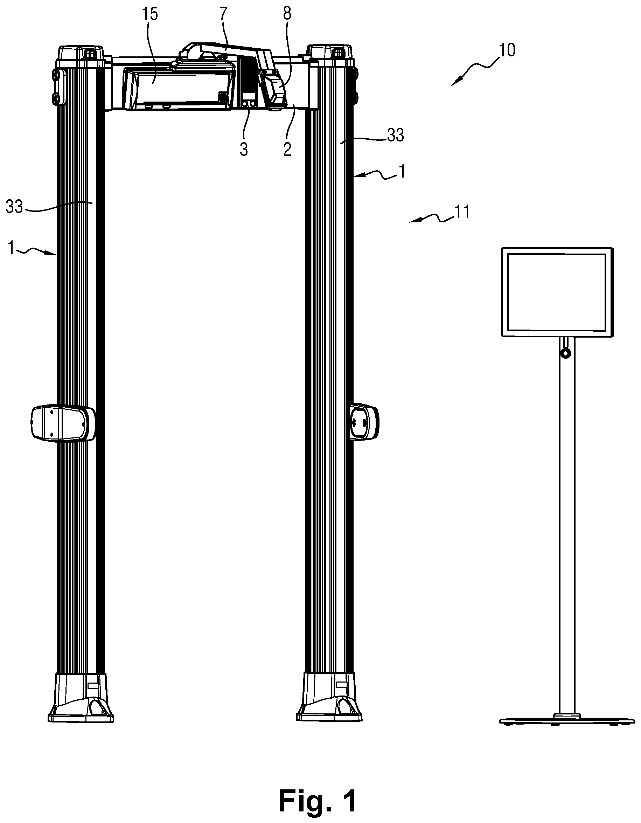

Dans une forme de réalisation, l'unité de traitement 15 est montée sur le même circuit imprimé que la puce de détection infrarouge 25. L'unité de traitement 15 peut notamment intégrer le processeur de la puce de détection infrarouge 25. En variante, l'unité de traitement 15 et le processeur de la caméra infrarouge 12 peuvent être distinctes, auquel cas l'unité de traitement 15 peut être logée dans le portique 11, à distance dans un pupitre séparé du portique 11 (voir par exemple figure 1) ou en réseau.

L'unité de traitement 15 est configurée pour déterminer la température corporelle d'un individu traversant le canal de passage 9 à partir de l'image l'image électronique obtenue par la caméra infrarouge 12 et, le cas échéant, à partir de l'image visible obtenue par la caméra à spectre visible 6.

Dans le cas où le système 10 comprend une caméra à spectre visible 6, l'unité de traitement 15 est connectée à la caméra à spectre visible 6 et configurée pour recevoir l'image visible et identifie dans cette image visible les pixels image visibles qui sont représentatif d'au moins une partie du visage d'un individu. Dans une forme de réalisation, l'unité de traitement 15 identifie de préférence une partie seulement du visage, typiquement les yeux voire un coin interne des yeux. En effet, la zone présentant la température la plus élevé dans un visage correspond généralement au coin interne de l'œil.

L'unité de traitement 15 est en outre connectée à la caméra infrarouge 12 et configurée pour recevoir l'image électronique et la mettre en correspondance avec l'image électronique de sorte à identifier dans l'image électronique les pixels images infrarouges qui correspondent aux pixels images visibles représentatifs du visage, ou le cas échéant aux yeux et/ou au coin interne d'un œil ou des yeux. Cette mise en correspondance permet en particulier de garantir que les valeurs de température calculées à partir de l'image électronique correspondent bien à des valeurs de température de l'individu, et non de son environnement. Le choix particulier des yeux, et plus particulièrement encore du coin interne des yeux, permet en outre de limiter les perturbations dues à l'environnement, en garantissant que la valeur de température mesurée est proche de la température corporelle de l'individu.

Enfin, quelle que soit la configuration (avec ou sans caméra à spectre visible 6), l'unité de traitement 15 est configurée pour déterminer une valeur de température maximale Tmax associée aux pixels images infrarouges correspondant au visage, aux yeux et/ou au coin interne des yeux, et pour en déduire la température corporelle de l'individu.

A titre de comparaison, les systèmes de mesure conventionnels, et notamment les systèmes portatifs (avec ou sans contact), ne permettent pas de réaliser une mesure de température dans le coin de l'œil, soit parce qu'ils sont conçus pour être mis en contact avec le front ou l'intérieur de l'oreille de l'individu, soit parce qu'il ne serait pas envisageable pour un opérateur de contrôler la température d'un individu à l'entrée d'une zone à accès limité en prenant une température au niveau de l'œil. Au contraire, l'invention réalisant une image électronique de l'individu, il est possible de déterminer dans l'image le point présentant la température la plus élevée, qui correspond généralement au coin interne de l'œil, sans contact et sans nécessiter l'intervention d'un opérateur.

Lorsque le système 10 ne comprend qu'une caméra infrarouge 12, 6, l'unité de traitement est configurée pour déterminer directement dans l'image électronique les pixels infrarouges électronique les pixels images infrarouges qui correspondent aux pixels images visibles représentatifs du visage, ou le cas échéant aux yeux et/ou au coin interne d'un œil ou des yeux.

Ainsi, seule la zone du visage, et de préférence seule la zone du visage comprenant les yeux et/ou le coin interne des yeux de l'individu, est en effet mesurée et c'est la température maximale Tmax dans cette zone limitée qui est comparée au seuil prédéterminé Tseuil. L'utilisation du système de mesure 10 permet ainsi de garantir l'obtention d'une valeur de température très proche voire égale à la température corporelle de l'individu.

## Capteur de présence 13

Afin de limiter les perturbations externes lors de la mesure de la température d'un individu, le système de mesure 10 peut en outre comprendre un capteur de présence 13 configuré pour déterminer la présence d'un individu au sein du canal de passage 9 du portique 11. L'unité de traitement 15 est alors configurée pour ne générer une image électronique et une image visible que lorsque le capteur de présence 13 détecte un objet (a priori un individu) au sein du canal de passage 9.

La Demanderesse s'est aperçue du fait que l'environnement dans lequel est positionné le système génère une énergie infrarouge susceptible de perturber les mesures de température. A titre d'exemple, il peut être noté qu'un éclairage de type DEL (pour diode électroluminescente) ou tube luminescent présente une température de l'ordre de 40°C, qui correspond à une température symptomatique de la fièvre et est donc susceptible de perturber la mesure de la température par le système. En ne générant l'image électronique que lorsque l'individu se trouve au sein du canal de passage 9, il est ainsi possible de garantir que l'individu occupe le champ visuel 31, 32 de la caméra infrarouge 12 et de la caméra à spectre visible 6 lors de la réalisation des images électronique et visible et que la mesure de la température ne soit ainsi pas perturbée par des éléments extérieurs.

Le capteur de présence 13 peut être fixé sur le portique 11, par exemple sur l'un des panneaux ou la traverse 2. Par exemple, le capteur de présence 13 peut comprendre une ou plusieurs barrières photoélectriques fixées sur les faces internes en regard des panneaux. De préférence, le système comprend au moins une barrière photoélectrique positionnée au niveau de l'entrée 9a du portique 11 et une barrière photoélectrique positionnée au niveau de la sortie 9b du portique 11 (et éventuellement une ou plusieurs barrières photoélectriques réparties entre les deux), l'unité de traitement 15 étant alors configurée pour générer une image électronique et une image visible entre le moment où la barrière photoélectrique située à l'entrée 9a et où la barrière photoélectrique à la sortie 9b détectent chacune la présence d'un individu.

De manière connue en soi, les barrières photoélectriques comprennent chacune un émetteur d'un faisceau lumineux, monté sur l'une des faces internes des panneaux latéraux 1, et un récepteur du faisceau lumineux, monté sur l'autre des faces internes. En variante, l'émetteur et le récepteur peuvent être fixés sur le même panneau latéral 1, la barrière photoélectrique comprenant alors un réflecteur fixé sur la face interne opposée et configurée pour réfléchir le signal lumineux émis par l'émetteur sur le réflecteur. Lors du passage d'un individu, la réception du faisceau lumineux par le récepteur est interrompue : une électronique d'évaluation (du type microprocesseur) envoie alors à l'unité de traitement 15 un signal électrique défini signalant le passage d'un individu. Lorsque la barrière photoélectrique est placée à l'entrée 9a du portique 11, l'unité centrale en déduit qu'un individu est présent dans le canal de passage 9. Lorsque la barrière photoélectrique est placée à la sortie 9b du portique 11, l'unité centrale en déduit que l'individu est sorti du canal de passage 9.

## Lumière 30

Optionnellement, le système de mesure 10 peut en outre comprendre une lumière 30, de préférence clignotante, positionnée à proximité de la caméra infrarouge 12 afin d'attirer le regard de l'individu au moment de la réalisation des images électronique et visible. Par exemple, la lumière 30 peut être montée sur le boîtier 8, à proximité de la caméra infrarouge 12.

Dans une forme de réalisation, la lumière 30 peut comprendre une diode électroluminescente DEL.

La lumière 30 peut être allumée, et le cas échéant clignoter, de manière continue. En variante, l'unité de traitement 15 peut être connectée à la lumière 30 de sorte à l'allumer, et le cas échéant la faire clignoter, uniquement lorsque l'image électronique et l'image visible doivent être réalisées. Typiquement, lorsque le système de mesure 10 comprend un capteur de présence 13, l'unité de traitement 15 peut allumer la lumière 30, et le cas échéant la faire clignoter, lorsqu'un individu est détecté par le capteur de présence 13.

## Unité de signalisation 33

Dans une forme de réalisation, le système de mesure 10 peut en outre comprendre au moins une unité de signalisation 33 configurée pour générer une alerte optique (signal lumineux) et/ou sonore (signal acoustique) lorsque la valeur de température maximale Tmax dépasse un seuil prédéterminé.

Optionnellement, l'unité de signalisation 33 peut également être configurée pour générer un signal lorsque la valeur de température maximale Tmax est inférieure ou égale au seuil prédéterminé Tseuil afin de signaler à un opérateur qu'une mesure a été effectuée mais que la température corporelle de l'individu est inférieure au seuil. A titre d'exemple, l'unité de signalisation 33 peut comprendre une lumière verte et une lumière rouge. L'unité de traitement 15 peut ainsi envoyer des instructions d'allumage de la lumière rouge lorsque la température maximale Tmax est supérieure au seuil prédéterminé Tseuil et de la lumière verte lorsqu'elle est inférieure ou égale à ce seuil prédéterminé.

## Procédé de mesure

La température corporelle d'un individu peut être mesurée à l'aide du système de mesure 10 conformément aux étapes suivantes.

Au cours d'une étape initiale S0, la présente d'un individu dans le portique 11 et un individu est déterminée par le capteur de présence 13. Pour cela, l'unité de traitement 15 interroge le capteur de présence 13, tel qu'une barrière photoélectrique.

Dans le cas d'un capteur de présence comprenant des barrières photoélectriques, lorsque la barrière située à l'entrée 9a (respectivement à la sorite) du portique 11 envoie un signal de présence à l'unité de traitement 15, celle-ci en déduit qu'un individu entre (respectivement sort) dans le canal de passage 9. L'unité de traitement 15 déclenche donc la mesure de température corporelle de l'individu entre la réception du signal de présence de la barrière située à l'entrée 9a et la réception du signal de présence de la barrière située à la sortie 9b.

**En** revanche, lorsque si les barrières photoélectriques, et notamment la barrière à l'entrée 9a, n'envoient pas de signal de présence, l'unité de traitement 15 en déduit qu'aucun individu ne se trouve dans le canal de passage 9 et ne déclenche donc pas la mesure de température.

Au cours d'une étape S1, l'unité de traitement 15 envoie des instructions à la caméra infrarouge 12 de réaliser une image électronique. Comme indiqué plus haut, la caméra infrarouge 12 est orientée, le cas échéant grâce au bras 7, de sorte que son champ visuel 32 couvre tout ou partie du canal de passage 9, dont au moins une portion supérieure.

L'image électronique étant réalisée entre les détections réalisées par la barrière à l'entrée 9a et la barrière à la sortie 9b du portique 11, elle comprend nécessairement l'individu. De plus, la caméra infrarouge 12 étant montée sur le portique 11 de sorte que son champ visuel 32 couvre au moins la portion supérieure du canal de passage 9, le visage et éventuellement le torse de l'individu se trouvent dans le champ visuel 32 de la caméra infrarouge 12.

Au cours d'une étape S2, l'unité de traitement 15 envoie des instructions à la caméra à spectre visible 6 de réaliser une image visible de tout ou partie de la portion du canal de passage 9.

Dans une forme de réalisation, le champ visuel 31 de la caméra à spectre visible 6 et le champ visuel 32 de la caméra infrarouge 12 couvrent sensiblement la même portion du canal de passage 9, et en tout état de cause la portion supérieure de celui-ci de sorte que l'image visible et l'image électronique couvrent toutes les deux le visage et le torse de l'individu.

Optionnellement, le champ visuel 32 de la caméra infrarouge 12 et/ou le champ visuel 31 de la caméra à spectre visible 6 peuvent couvrir toute la hauteur du canal de passage 9.

De préférence, les étapes S1 et S2 sont simultanées, afin de faciliter la mise en correspondance des images visible et infrarouge. Par ailleurs, dans une forme de réalisation, la lumière 30 peut être allumée par l'unité de traitement 15 (ou être allumée de manière continue), le cas échéant de façon à clignoter, afin d'attirer le regard de l'individu au moment de la réalisation des images électronique et visible au cours des étapes S1 et S2.

Au cours d'une étape S3, l'unité de traitement 15 identifie dans l'image visible les pixels image visibles qui correspondent au visage de l'individu, aux yeux de l'individu et/ou aux coins internes des yeux. De préférence, l'unité de traitement 15 identifie les pixels image visibles correspondant à au moins un coin interne des yeux.

Pour cela, l'unité de traitement 15 détecte le visage (respectivement, les yeux et/ou au moins un coin interne des yeux) suivant la méthode de Viola et Jones (ou image intégrale), qui est un procédé d'apprentissage supervisé utilisant un classifieur en cascade de caractéristiques de Haar. On pourra se référer pour plus de détails à l'article de Paul Viola et Michael Jones, « Rapid Object Detection using a Boosted Cascade of Simple Features », 2001 IEEE Computer Society Conference on Computer Vision and Pattern Recognition, pour plus de détails sur cette méthode.

D'autres méthodes peuvent être utilisées pour la réalisation de l'étape S3, telles que des méthodes de Deep Learning (apprentissage profond en français) utilisant un classifieur de segmentation sémantique. On pourra notamment se référer à l'article de Alex Krizhevsky, Ilya Stuskever et Geoffroy E. Hinton, « ImageNet Classification with Deep Convolutional Neural Networks » ou à l'article de Vijay Badrinarayanan, Alex Kendall et Roberto Cipolla, « SegNet : A Deep Convolutional Encoder-Decoder Architecture for Image Segmentation », pour plus de détails sur l'utilisation de classifieurs de segmentation sémantique. Ces méthodes peuvent le cas échéant être utilisées et combinées pour augmenter la vitesse de détection et la précision des caractéristiques recherchées.

Au cours d'une étape S4, l'unité de traitement 15 met en correspondance l'image visible et l'image électronique de sorte à identifier les pixels images infrarouges correspondant aux pixels images visibles identifiés à l'étape S3.

La mise en correspondances des pixels images visibles et infrarouges peut être réalisée par transposition des coordonnées des pixels images de l'image visible à l'image électronique en tenant compte des positions et orientations de la caméra à spectre visible 6 et de la caméra infrarouge 12, de manière connue pour l'homme du métier. Par exemple, cela peut se faire par apprentissage des caractéristiques des caméras 6, 12 de manière automatique (paramètres intrinsèques aux caméras 6, 12 comme la focale et la distorsion, et paramètres extrinsèques comme la position et l'orientation). Cet apprentissage réalisé une fois pour toutes, typiquement lors de l'installation et la calibration du système de mesure 10. Par exemple, la transposition peut être faite par mise en œuvre d'une application affine de l'image visible dans l'image électronique. A cet effet, les coefficients de l'application affine sont déterminés lors de la calibration du système de mesure 10. Pour cela, des références visuelles peuvent être positionnées dans l'espace à des positions connues par rapport aux, puis des images visible et infrarouge de ces références visuelles sont réalisées avec les caméras 6, 12. Connaissant la position tridimensionnelle des références visuelles par rapport aux deux caméras 6, 12, il est alors possible de déterminer les coefficients de l'application affine de l'image visible dans l'image électronique.

Au cours d'une étape S9, l'unité de traitement 15 détermine la valeur de température maximale Tmax associée aux pixels image infrarouges identifiés à l'étape S4 et en déduit la température corporelle de l'individu.

## Calibration des mesures infrarouges

Selon l'invention, pour corriger l'image électronique obtenue par la caméra infrarouge 12 et assurer la fiabilité de la mesure de la température corporelle, le système de mesure 10 comprend en outre :

- un support 16 ;
- un premier étalon 4 et un deuxième étalon 5 positionnés dans le champ visuel 32 de la caméra infrarouge 12 ; et
- une première sonde thermique 20 et une deuxième sonde thermique 22 configurées pour mesurer une température instantanée du premier étalon 4 et du deuxième étalon 5.

Par ailleurs, l'unité de traitement 15 est en outre configurée pour déterminer un coefficient de gain et un coefficient de décalage de la caméra infrarouge 12 et corriger ainsi l'image électronique obtenue par la caméra infrarouge 12, en vue de déterminer à l'étape S9 la valeur de température maximale Tmax associées aux pixels images infrarouges identifiés à l'étape S4.

Dans ce qui suit, l'invention sera décrite de manière générale dans le cas où l'unité de traitement 15 réalise la calibration des mesures infrarouges. Ceci n'est cependant pas limitatif, cette calibration pouvant être réalisée par tout processeur ou microprocesseur, tel qu'une puce microcontrôleur dédiée qui est contrôlée par l'unité de traitement 15 ou par un ordinateur extérieur local ou à distance en réseau.

## Premier étalon 4 et deuxième étalon 5

Le premier étalon 4 présente une première température de référence et le deuxième étalon 5 présente une deuxième température de référence et ont pour fonction de permettre la calibration de la caméra infrarouge 12 lors de la réalisation de chaque image électronique. La première température de référence et la deuxième température de référence sont de préférence différentes. Le premier étalon 4 et le deuxième étalon 5 peuvent par exemple chacun comprendre une surface, chauffée à la température de référence correspondante. Le premier étalon 4 et le deuxième étalon 5 peuvent par exemple chacun comprendre une surface, qui peut être sensiblement plane, chauffée à la température de référence correspondante.

Afin de garantir une correction optimale de l'image électronique réalisée par la caméra infrarouge 12, la première et la deuxième température sont proches de la température corporelle à partir de laquelle on considère habituellement qu'un individu présente des symptômes de fièvre, typiquement 37,5°C. La première et la deuxième température de référence peuvent par exemple être comprises entre 35°C et 40°C. Dans un exemple de réalisation, la première température de référence est égale à 36°C et la deuxième température de référence est égale à 39°C.

Le premier étalon 4 et le deuxième étalon 5 sont chacun positionnés dans le champ visuel 32 de la caméra infrarouge 12 de sorte que l'image électronique comprend des pixels image représentatifs de leur valeur de température respective. De préférence, le premier étalon 4 et le deuxième étalon 5 sont fixes par rapport à la caméra infrarouge 12 et sont placés dans le champ visuel 32 de la caméra infrarouge 12.

Le premier étalon 4 et le deuxième étalon 5 peuvent par exemple être fixés sur le portique 11. Dans un exemple de réalisation, le premier étalon 4 et le deuxième étalon 5 sont fixés sur la traverse 2 du portique 11, dans le champ visuel 32 de la caméra infrarouge 12. Cette configuration est notamment envisageable lorsque la caméra infrarouge 12 est montée sur un bras 7 de sorte à s'étendre en aval du canal de passage 9. Avantageusement, la fixation des premier et deuxième étalons 4, 5 sur la traverse 2 permet de garantir que les étalons 4, 5 soient fixes par rapport à la caméra infrarouge 12 et ne risquent pas d'être masqués par l'individu lors de son passage au travers du canal.

Le cas échéant, le premier étalon 4 et le deuxième étalon 5 peuvent être logé dans un même boîtier 3.

La position et la surface du premier étalon 4 et du deuxième étalon 5 par rapport à la caméra infrarouge 12 sont choisies de sorte que le premier étalon 4 et le deuxième étalon 5 s'étendent chacun dans le champ visuel 32 d'au moins un pixel de la puce de détection infrarouge 25, par exemple d'une une sous-matrice comprenant 3x3 pixels. Pour cela, le premier et le deuxième étalon 4, 5 peuvent être placés à une distance entre 20 cm et 1.5 mètres de la puce de détection infrarouge 25 et ont une surface comprise entre 25 $mm^2$ et 400 $cm^2$.

Dans une variante de réalisation, le premier étalon 4 et le deuxième étalon 5 peuvent être fixés sur la face interne de l'un et/ou l'autre des panneaux latéraux 1 du portique 11, de préférence à proximité de la traverse 2.

Optionnellement, afin de protéger le premier et le deuxième étalon 4, 5, le système peut en outre comprendre un diaphragme 17, 18 positionné devant chaque étalon 4, 5 afin de les protéger de l'environnement et d'éviter des perturbations éventuelles (telles que la présence d'un courant d'air ou tout élément susceptible de modifier la température des étalons 4, 5). Le cas échéant, le ou les diaphragmes 17, 18 peuvent être montés entre le premier étalon 4 et le deuxième étalon 5, respectivement, et un couvercle du boîtier 3.

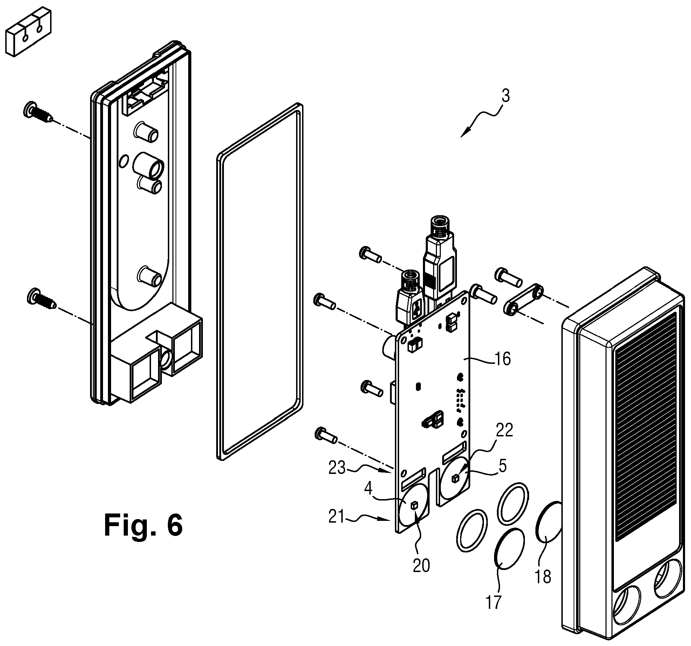

Le système de mesure 10 comprend en outre un premier élément de chauffe 21 et un deuxième élément de chauffe 23 configurés pour maintenir le premier étalon 4 et le deuxième étalon 5 à la première température de référence et à la deuxième température de référence, respectivement. Le premier et le deuxième élément de chauffe 21, 23 peuvent par exemple chacun comprendre une résistance raccordée au premier et au deuxième étalon 4, 5, respectivement. Dans une forme de réalisation, le premier et le deuxième élément de chauffe 21, 23 sont monté sur la face opposée à la caméra infrarouge 12 (qui correspond à la face opposée aux premier et deuxième étalons 4, 5) de sorte à ne pas être vus par la caméra infrarouge 12 (voir figure 6).

Le cas échéant, le premier et le deuxième élément de chauffe 21, 23 peuvent être logés dans le même boîtier 3 que le premier et le deuxième étalon 4, 5.

Première et deuxième sondes thermiques 20, 22

La première et la deuxième sonde thermiques 20, 22 sont configurées pour mesurer une température instantanée du premier étalon 4 et du deuxième étalon 5, respectivement. Selon l'invention, la première et la deuxième sonde thermique 20, 22 sont en contact (direct ou indirect) avec le premier ou le deuxième étalon 4, 5, respectivement. La première et la deuxième sonde thermique 20, 22 sont de préférence monolithiques avec le premier et le deuxième étalon 4, 5, respectivement. On notera toutefois que, de préférence, la première est la deuxième sonde thermique 20, 22 sont de préférence positionnées de sorte à ne pas former un obstacle entre l'individu et la caméra infrarouge 12.

De préférence, la première et la deuxième sonde thermique 20, 18 présentent une précision de mesure inférieure ou égale à 0.1°C afin de fournir une valeur très précise de la température instantanée de chaque étalon 4, 5. Comme nous le verrons par la suite, c'est cette valeur de la température instantanée du premier et du deuxième étalon 4, 5 qui est ensuite utilisée par l'unité de traitement 15, et non la valeur de la première et la deuxième température de référence programmées, pour corriger l'image électronique générée par la caméra infrarouge 12.

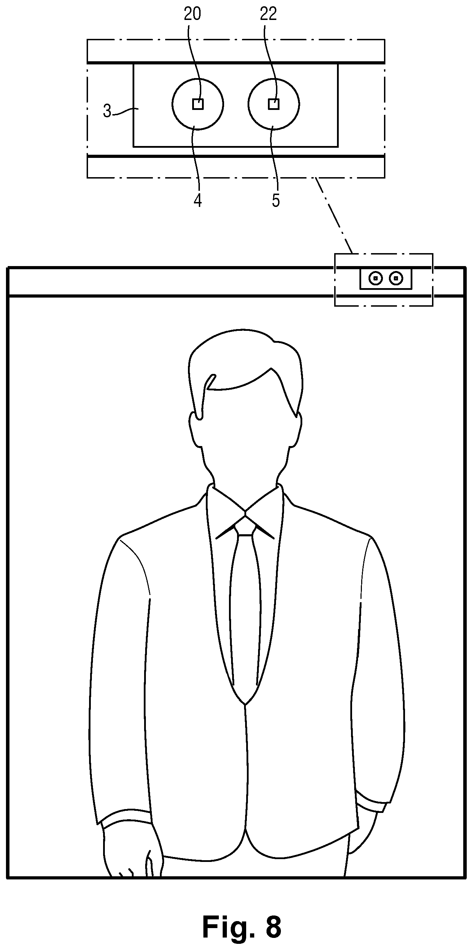

Selon l'invention, la sonde thermique 20, 22 est connectée à l'étalon 4, 5 correspondant par l'intermédiaire d'une piste conductrice. La Demanderesse s'est cependant aperçue du fait que, en fonctionnement, la température de cette piste conductrice était sensiblement égale (à 0.1 °C près) à la température de la surface chauffée de l'étalon 4, 5 correspondant. Par conséquent, dans une forme de réalisation, la piste conductrice qui connecte la première sonde thermique 20 à la surface chauffée du premier étalon 4 et la deuxième sonde thermique à la surface chauffée du deuxième étalon 5 peut être considérée comme faisant partie des étalons 4, 5 respectivement par d'unité de traitement 15. Dans cette forme de réalisation, la première et la deuxième sonde thermique 20, 22 peuvent donc être montées à proximité immédiate du premier étalon 4 et du deuxième étalon 5. Selon l'invention, la première sonde thermique 20 peut être fixée au centre de la surface chauffée du premier étalon 4 et la deuxième sonde thermique 22 est fixée au centre de la surface chauffée du deuxième étalon 5 (voir par exemple figure 8). La première et la deuxième sonde thermique 20, 22 peuvent par exemple comprendre une puce semi-conductrice. L'étalon 4, 5 est alors encadré par la partie supérieure de la puce semi-conductrice, au bord de la zone de lecture.

Lorsque le support 16 comprend un circuit imprimé supplémentaire, les sondes thermiques peuvent être montées sur ce circuit imprimé supplémentaire.

Le cas échéant, le premier et le deuxième élément de chauffe 21, 23 peuvent être commandés par l'unité de traitement 15 en fonction de la valeur instantanée de la température du premier et du deuxième étalon 4, 5 qui est mesurée par la première et la deuxième sonde thermique 20, 22.

Afin de corriger l'image électronique de la caméra infrarouge 12, l'unité de traitement 15 est en outre configurée pour déterminer le coefficient de gain et le coefficient de décalage à partir des températures instantanées respectives du premier étalon 4 et du deuxième étalon 5 et des valeurs de température du premier étalon 4 et du deuxième étalon 5 dans l'image électronique et pour appliquer le coefficient de gain et le coefficient de décalage ainsi déterminés à la valeur de température associée à chaque pixel image de sorte à obtenir l'image électronique corrigée.

Ainsi, contrairement aux dispositifs de mesure conventionnels, le système de mesure 10 réalisant une image électronique de l'individu et déterminant la valeur de température au niveau de chaque pixel image, il ne se contente pas de la prise de mesure en un point aléatoire du visage de l'individu et limite ainsi les risques de perturbations de la mesure par l'environnement.

L'unité de traitement 15 est donc configurée pour exécuter des instructions et contrôler la première et la deuxième sonde thermique 20, 22 et le premier et le deuxième élément de chauffe 21 et 23.

Procédé de calibration des mesures infrarouges

Les valeurs de température associées aux pixels image infrarouge identifiés à l'étape S4 peuvent ensuite être calibrée à l'aide du système de mesure 10 conformément aux étapes suivantes.

Au cours d'une étape S5, la température instantanée du premier étalon 4 et du deuxième étalon 5 est déterminée par la première sonde thermique 20 et la deuxième sonde thermique 22, respectivement.

De préférence, les étapes S1 et S5 sont simultanées. Par simultanées, on comprendra ici que les étapes S1 et S5 sont réalisées en même temps, ou avec un décalage temporel au plus égal au temps nécessaire pour que la température du premier étalon 4 et du deuxième étalon 5 change de 0.1 °.

Au cours d'une étape S6, l'unité de traitement 15 détermine dans l'image électronique obtenue à l'étape S1 la valeur de température de chaque pixel image infrarouge correspondant au premier étalon 4 et au deuxième étalon 5. Les pixels images infrarouges correspondant aux étalons 4, 5 peuvent être identifiés dans l'image électronique dans la mesure où la position spatiale des premier et deuxième étalons 4, 5 par rapport à la caméra infrarouge 12 est connue et fixe.

Selon l'invention, notamment lorsque les sondes thermiques comprennent une puce semi-conductrice, l'unité de traitement 15 détermine plus particulièrement la valeur de température de chaque pixel image infrarouge correspondant à la piste conductrice connectant le premier étalon 4 et le deuxième étalon 5 à la première sonde thermique 20 et à la deuxième sonde thermique 22. Comme nous l'avons vu plus haut, ces pistes conductrices ont en effet une température sensiblement égale à celle de l'étalon 4, 5 correspondant et sont donc considérée comme faisant partie du premier étalon 4 et du deuxième étalon 5, respectivement. La zone de superposition des sondes thermiques 20, 22 et de la surface chauffée des étalons 4, 5 permet en effet de réduire d'une manière significative les erreurs causées par les chutes thermiques. Dans une variante ne correspondant pas à l'invention, l'unité de traitement 15 peut déterminer la valeur de température associée à la surface chauffée des étalons 4, 5. Lorsque plusieurs pixels images correspondent au premier étalon 4 et au deuxième étalon 5 dans l'image électronique, l'unité de traitement 15 peut notamment choisir la valeur de température moyenne parmi la sous-matrice de pixels images comme valeur instantanée de l'étalon 4, 5 correspondant.

Au cours d'une étape S7, l'unité de traitement 15 déduit des valeurs de température déterminées à l'étape S6 et de la température instantanée des premier et deuxième étalon 4 et 5 mesurée à l'étape S5 un coefficient de gain $k_1$ et un coefficient de décalage $k_2$ de la caméra infrarouge 12. Le coefficient de gain $k_1$ correspond à une déviation en amplitude de la caméra infrarouge 12 tandis que le coefficient de décalage $k_2$ correspond à une erreur ayant une valeur constante correspondant à un décalage des valeurs mesurées sur l'axe des ordonnées de la tension de sortie de la puce de détection infrarouge 25.

Plus précisément, la température instantanée $T_{i\_1}$ (respectivement, $T_{i\_2}$) du premier étalon 4 (respectivement, du deuxième étalon 5) est égale à la somme du coefficient de gain $K_1$ multiplié par la valeur de température $T_{IR\_1}$ (respectivement, $T_{IR\_2}$) déterminée à l'étape S3 pour le premier étalon 4 (respectivement, pour le deuxième étalon 5) et du coefficient de décalage $k_2$ :

$$T_{i\_1} = T_{IR\_1} * k_1 + k_2$$

$$T_{i\_2} = T_{IR\_2} * k_1 + k_2$$

La température instantanée $T_{i\_1}$, $T_{i\_2}$ du premier étalon 4 et du deuxième étalon 5 sont indépendantes de la déviation de la caméra infrarouge 12, étant mesurée par la première et la deuxième sonde thermique 20, 22. Par ailleurs, la déviation de la caméra infrarouge 12 est la même pour les valeurs de température $T_{IR\_1}$ et $T_{IR\_2}$ déterminées à l'étape S3. Il en découle que $k_1$ et $k_2$ sont identiques dans ces deux équations. Ainsi, la résolution de ces équations, permise par la présence des deux étalons 4, 5 et la détermination de leur température instantanée $T_{i\_1}$ et $T_{i\_2}$ par les sondes thermiques 20, 22 associées, permet de déterminer la valeur des coefficients de gain $k_1$ et de décalage $k_2$ de la caméra infrarouge 12.

Au cours d'une étape S5, l'unité de traitement 15 applique le coefficient de gain $k_1$ et le coefficient de décalage $k_2$ déterminés à l'étape S4 à la valeur de température associée à chaque pixel image infrarouge de sorte à obtenir une image électronique corrigée.

Ainsi, pour tout pixel image infrarouge i de l'image électronique générée à l'étape S3, l'unité de traitement 15 applique le coefficient de gain $k_1$ et le coefficient de décalage $k_2$ à la valeur de température $T_{IR\_i}$ associée à ce pixel image infrarouge i de sorte à obtenir, pour chaque pixel image i, la valeur de température corrigée $T_{corr\_i}$ et en déduire l'image électronique corrigée qui comprend les pixel images infrarouges corrigés :

$$T_{corr\_i} = k_1 * + T_{IR\_i} + k_2$$

Au cours de l'étape S9, l'unité de traitement 15 détermine donc la valeur de température maximale Tmax dans l'image électronique corrigée et compare cette valeur de température maximale Tmax avec un seuil prédéterminé Tseuil. La température corporelle à partir de laquelle on considère habituellement qu'un individu présente des symptômes de fièvre étant généralement de 37,5°C, le seuil prédéterminé Tseuil peut par exemple être égal à 37,5°C.

Lorsque la température maximale Tmax est supérieure ou égale au seuil prédéterminé Tseuil, l'unité de traitement 15 envoie des instructions à l'unité de signalisation 33 afin de générer une alerte (typiquement, une alerte visuelle et/ou acoustique).

Dans une forme de réalisation, le procédé de mesure comprend en outre une étape S8 au cours de laquelle l'unité de traitement 15 applique un coefficient de compensation $k_3$ prédéterminé à la valeur de température de chaque pixel image infrarouge afin de compenser une différence d'émissivité entre les premier et deuxième étalons 4, 5 et la peau humaine et obtenir ainsi une valeur de température corrigée plus proche de la valeur réelle de la température corporelle de l'individu :

$$T_{finale\_i} = T_{corr\_i} * k_3$$

C'est alors cette valeur de température corrigée et compensée $T_{final\_i}$ qui est utilisée au cours de l'étape S9 pour déterminer la valeur de température maximale Tmax, et non la valeur corrigée $T_{corr\_i}$.

Dans une variante de réalisation, le coefficient de compensation peut n'être appliqué qu'à la valeur maximale de température Tmax déterminée à l'étape S9. L'étape S8 a alors lieu après l'étape S9.

Le coefficient de compensation $k_3$ est un coefficient fixe, prédéterminé, qui ne dépend pas d'une éventuelle déviation thermique ni de l'environnement de mesure. Ce coefficient dépend en revanche de l'émissivité de la peau humaine et de l'émissivité des premier et deuxième étalons 4, 5, et plus particulièrement du matériau constitutif de leur surface chauffée ou, le cas échéant, de la piste conductrice qui y est connectée. C'est pourquoi il peut être préférable de réaliser le premier et le deuxième étalon 4, 5 dans un même matériau constitutif, seule leur température de référence respective étant différente.

Dans une forme de réalisation, les étapes S1 à S9 peuvent être mises en œuvre de manière continue suivant la même période d'interrogation (qui peut être comprise entre dix millisecondes et cinq cent millisecondes d'interrogation), indépendamment de la détection d'un individu dans le canal de passage 9. Dans ce cas, l'unité de traitement 15 n'envoie des instructions de génération d'une alerte à l'unité de signalisation 33 que si le capteur de présence 13 détecte un individu dans le canal de passage 9.

De plus, les étapes S1 à S9 sont répétées tant que l'individu est dans le canal de passage 9, typiquement tant que la barrière photoélectrique située à la sortie 9b du canal de passage 9 n'a pas détecté la sortie de l'individu. La période de répétition de ces étapes peut notamment être égale à la période d'interrogation du capteur de présence 13 par l'unité de traitement 15.

Par ailleurs, on notera qu'à tout moment pendant le fonctionnement du système (et donc pendant l'interrogation du capteur de présence 13 par l'unité de traitement 15), les premier et deuxième éléments de chauffe 21, 23 maintiennent le premier étalon 4 et le deuxième étalon 5 à la première température de référence et à la deuxième température de référence, respectivement, de sorte à garantir que la température instantanée desdits éléments soit proche de leur température de référence respective lors des étapes S1 et S5.

De manière connue en soi, le système de mesure 10 comprend en outre un kiosque comprenant par une embase et un écran, connecté à l'unité de traitement du système, par exemple par l'intermédiaire d'une interface Ethernet.

## Revendications

**1.** Système de mesure (10) d'une température corporelle d'un individu comprenant :

un portique (11) comprenant deux panneaux latéraux (1) reliés par une traverse (2) et délimitant ensemble un canal de passage (9) pour un individu ;

une caméra infrarouge (12) comprenant une puce de détection infrarouge (25) comprenant une matrice de pixels infrarouges et étant configurée pour convertir un rayonnement infrarouge reçu par chaque pixel infrarouge en une valeur de température correspondante et pour générer une image électronique comprenant une pluralité de pixels images infrarouges, chaque pixel image infrarouge étant représentatif de la valeur de température reçue par un pixel infrarouge correspondant, la caméra infrarouge (12) étant fixée sur le portique (11) de sorte qu'un champ visuel (32) de la caméra infrarouge (12) couvre tout ou partie de la portion du canal de passage (9) ;

une unité de traitement (15) configurée pour identifier les pixels infrarouges correspondant à au moins une partie d'un visage d'un individu et pour déterminer une valeur de température maximale associée aux pixels infrarouges identifiés et pour en déduire une température corporelle de l'individu ; et

un module de calibration (3) comprenant un premier étalon (4) et un deuxième étalon (5) positionnés dans le champ visuel (32) de la caméra infrarouge (12) de sorte que l'image électronique comprend des pixels image infrarouge représentatifs de la valeur de température du premier étalon (4) et du deuxième étalon (5), le module de calibration comprend en outre une première sonde thermique (20) fixée au centre d'une surface chauffée du premier étalon (4) et configurée pour mesurer une température instantanée du premier étalon (4) et une deuxième sonde thermique (22) fixée au centre d'une surface chauffée du deuxième étalon (5) et configurée pour mesurer une température instantanée du deuxième étalon (5), la première et la deuxième sonde thermique (20, 22) étant raccordées au premier et au deuxième étalon (4, 5), respectivement, par l'intermédiaire d'une première et d'une deuxième piste conductrice,

l'unité de traitement (15) est en outre configurée pour :

déterminer une température instantanée de la première et de la deuxième piste conductrice et en déduire la température instantanée du premier et du deuxième étalon (4, 5) respectivement ; et

déterminer un coefficient de gain (k₁) et un coefficient de décalage (k₂) à partir des températures instantanées respectives du premier étalon (4) et du deuxième étalon (5) et des valeurs de température du premier étalon (4) et du deuxième étalon (5) dans l'image électronique et pour appliquer le coefficient de gain (k₁) et le coefficient de décalage (k₂) ainsi déterminés à la valeur de température associée à chaque pixel image infrarouge de sorte à obtenir une image électronique corrigée, l'unité de traitement (15) étant configurée pour déterminer la valeur de température maximale à partir de l'image électronique corrigée.

2. Système de mesure (10) selon la revendication 1, comprenant en outre une caméra à spectre visible (6) comprenant une puce de détection de spectre visible (29) comprenant une matrice de pixels visibles et étant configurée pour générer une image visible comprenant une pluralité de pixels images visibles, la caméra à spectre visible (6) étant fixée sur le portique (11) de sorte qu'un champ visuel (31) de la caméra à spectre visible (6) couvre au moins une portion du canal de passage (9), l'unité de traitement étant configurée pour mettre en correspondance l'image visible et l'image électronique de sorte à identifier les pixels images infrarouges correspondant aux pixels images visibles identifiés.

3. Système de mesure (10) selon l'une des revendications 1 à 2, dans lequel l'unité de traitement (15) est en outre configurée pour appliquer un coefficient de compensation (k₃) prédéterminé à la valeur de température de chaque pixel image infrarouge.

4. Système de mesure (10) selon l'une des revendications 1 à 3, dans le lequel la première et la deuxième sonde thermique (20, 22) comprennent chacune une puce semi-conductrice raccordée au premier et au deuxième étalon (4, 5), respectivement, par l'intermédiaire de la première et de la deuxième piste conductrice.

5. Système de mesure selon l'une des revendications 1 à 4, dans lequel dans lequel le premier étalon (4) et le deuxième étalon (5) sont fixés sur l'un parmi les deux panneaux latéraux (1) et la traverse (2).

6. Système de mesure (10) selon l'une des revendications 1 à 5, comprenant en outre un capteur de présence (13) configuré pour déterminer une présence d'un individu dans le canal de passage (9), l'unité de traitement (15) étant configurée pour ne générer l'image électronique que lorsque le capteur de présence (13) détecte un objet au sein du canal de passage (9).

7. Système de mesure (10) selon la revendication 2, dans lequel la caméra infrarouge (12) et la caméra à spectre visible (6) sont montées sur un bras (7) fixé sur la traverse (2) et s'étendant depuis une sortie (9b) du portique (11).

8. Système de mesure (10) selon l'une des revendications 1 à 7, comprenant en outre une lumière (30), de préférence clignotante, fixée à proximité de la caméra infrarouge (12) de sorte à attirer un regard d'un individu transitant dans le canal de passage (9).

9. Système de mesure (10) selon l'une des revendications 1 à 8, dans lequel Le champ visuel (32) de la caméra infrarouge (12) présente par un angle vertical (φ) et un angle horizontal (θ) supérieurs ou égal 30° et inférieur ou égal à 120°.

10. Procédé de mesure d'une température corporelle d'un individu à l'aide d'un système de mesure (10) selon l'une des revendications 1 à 9, comprenant les étapes suivantes :

S1 : réaliser une image électronique d'une portion du canal de passage (9) dans lequel se trouve un individu, l'image électronique comprenant une pluralité de pixels images infrarouges représentatifs d'une valeur de température reçue par un pixel infrarouge correspondant d'une matrice de pixels infrarouge d'une caméra infrarouge (12), un premier étalon (4) et un deuxième étalon (5) étant positionnés dans le champ visuel (32) de la caméra infrarouge (12) de sorte que l'image électronique comprend des pixels image infrarouge représentatifs de la valeur de température du premier étalon (4) et du deuxième étalon (5) ;

S4 : identifier les pixels images infrarouge correspondant à au moins une partie d'un visage de l'individu, de préférence à au moins un coin interne des yeux ;

S5 : déterminer une température instantanée d'une piste conductrice d'une première sonde thermique (20) fixée au centre d'une surface chauffante du premier étalon (4) et d'une piste conductrice d'une deuxième sonde thermique (22) fixée au centre d'une surface chauffante du deuxième étalon (5) ;

S6 : déterminer dans l'image électronique la valeur de température des pixels images correspondant au premier étalon (4) et au deuxième étalon (5) ;

S7 : en déduire un coefficient de gain (k₁) et un coefficient de décalage (k₂) de la caméra infrarouge (12) ; et

S8 : appliquer le coefficient de gain (k₁) et le coefficient de décalage (k₂) à la valeur de température associée à chaque pixel image de sorte à obtenir une image électronique corrigée ; et
S9 : déterminer une valeur de température maximale associée aux pixels image infrarouges identifiés et en déduire une température corporelle de l'individu.

11. Procédé de mesure selon la revendication 10, comprenant en outre les étapes suivantes, préalablement à l'étape S9 :

S2 : réaliser une image visible de tout ou partie de la portion du canal de passage (9), l'image visible comprenant une pluralité de pixels images visibles;
S3 : identifier les pixels image visibles correspondant à au moins une partie d'un visage de l'individu, de préférence au moins un coin interne des yeux ; et
de sorte que l'étape S4 comprend la mise en correspondance de l'image visible et de l'image électronique de sorte à identifier les pixels images infrarouges correspondant aux pixels images visibles identifiés à l'étape S3,
les étapes S1 et S2 pouvant être simultanées.

12. Procédé selon l'une des revendications 10 ou 11, comprenant l'application d'un coefficient de compensation (k₃) prédéterminé à la valeur de température de chaque pixel image ou à la valeur de température maximale (Tmax) déterminée à l'étape S9.

13. Procédé selon l'une des revendications 10 à 12, comprenant en outre, préalablement à l'étape S1, une étape S0 de détermination d'une présence d'un individu dans le portique (11), les étapes S1 à S9 n'étant mises en œuvre que lorsqu'un individu est présent dans le canal de passage (9).

**Patentansprüche**

1. System (10) zum Messen einer Körpertemperatur eines Individuums, umfassend:

ein Portal (11), das zwei Seitenwände (1) umfasst, die durch einen Querträger (2) verbunden sind und zusammen einen Durchgangskanal (9) für ein Individuum begrenzen;
eine Infrarotkamera (12), die einen Infrarotdetektionschip (25) umfasst, der eine Infrarotpixelmatrix umfasst und ausgelegt ist, um eine von jedem Infrarotpixel empfangene Infrarotstrahlung in einen entsprechenden Temperaturwert umzuwandeln und um ein elektronisches Bild zu erzeugen, das eine Vielzahl von Infrarotbildpixeln umfasst, wobei jedes Infrarotbildpixel für den von einem entsprechenden Infrarotpixel empfangenen Temperaturwert repräsentativ ist, wobei die Infrarotkamera (12) an dem Portal (11) derart befestigt ist, dass ein Sichtfeld (32) der Infrarotkamera (12) den gesamten oder einen Teil des Abschnitts des Durchgangskanals (9) abdeckt;
eine Verarbeitungseinheit (15), die ausgelegt ist, um die Infrarotpixel zu identifizieren, die mindestens einem Teil eines Gesichts eines Individuums entsprechen, und um einen maximalen Temperaturwert zu bestimmen, der den identifizierten Infrarotpixeln zugeordnet ist, und um daraus eine Körpertemperatur des Individuums abzuleiten; und
ein Kalibrierungsmodul (3), das ein erstes Richtmaß (4) und ein zweites Richtmaß (5) umfasst, die im Sichtfeld (32) der Infrarotkamera (12) derart positioniert sind, dass das elektronische Bild Infrarotbildpixel umfasst, die für den Temperaturwert des ersten Richtmaßes (4) und des zweiten Richtmaßes (5) repräsentativ sind,
wobei das Kalibrierungsmodul ferner eine erste Wärmesonde (20) umfasst, die in der Mitte einer Heizfläche des ersten Richtmaßes (4) befestigt und konfiguriert ist, um eine momentane Temperatur des ersten Richtmaßes (4) zu messen, und eine zweite Wärmesonde (22), die in der Mitte einer Heizfläche des zweiten Richtmaßes (5) befestigt und konfiguriert ist, um eine momentane Temperatur des zweiten Richtmaßes (5) zu messen, wobei die erste und die zweite Wärmesonde (20, 22) jeweils über eine erste und eine zweite Leiterbahn mit dem ersten bzw. dem zweiten Richtmaß (4, 5) verbunden sind,
wobei die Verarbeitungseinheit (15) ferner ausgelegt ist, um:

eine momentane Temperatur der ersten und der zweiten Leiterbahn zu bestimmen und daraus die momentane Temperatur jeweils des ersten und des zweiten Richtmaßes (4, 5) abzuleiten; und
einen Verstärkungskoeffizienten (k₁) und einen Verschiebungskoeffizienten (k₂) anhand der jeweiligen Momentantemperaturen des ersten Richtmaßes (4) und des zweiten Richtmaßes (5) und den Temperaturwerten des ersten Richtmaßes (4) und des zweiten Richtmaßes (5) in dem elektronischen Bild abzuleiten und um den derart bestimmten Verstärkungskoeffizienten (k₁) und den Verschiebungskoeffizienten (k₂) auf den jedem Infrarotbildpixel zugeordneten Temperaturwert derart anzuwenden, dass ein korrigiertes elektronisches Bild erhalten wird, wobei die Verarbeitungseinheit (15) ausgelegt ist, um den maximalen Temperaturwert anhand des korrigierten elektronischen Bildes zu bestimmen.

2. Messsystem (10) nach Anspruch 1, das ferner eine Kamera (6) für sichtbares Spektrum umfasst, die einen Detektionschip (29) für sichtbares Spektrum umfasst, der eine Matrix sichtbarer Pixel umfasst und ausgelegt ist, um ein sichtbares Bild zu erzeugen, das eine Vielzahl sichtbarer Bildpixel umfasst, wobei die Kamera für sichtbares Spektrum (6) derart an dem Portal (11) befestigt ist, dass ein Sichtfeld (31) der Kamera für sichtbares Spektrum (6) mindestens einen Abschnitt des Durchgangskanals (9) abdeckt, wobei die Verarbeitungseinheit ausgelegt ist, um das sichtbare Bild und das elektronische Bild abzugleichen, um die Infrarotbildpixel zu identifizieren, die den identifizierten sichtbaren Bildpixeln entsprechen.

3. Messsystem (10) nach einem der Ansprüche 1 bis 2, wobei die Verarbeitungseinheit (15) ferner ausgelegt ist, um einen vorbestimmten Kompensationskoeffizienten ($k_3$) auf den Temperaturwert jedes Infrarotbildpixels anzuwenden.

4. Messsystem (10) nach einem der Ansprüche 1 bis 3, wobei die erste und die zweite Temperatursonde (20, 22) jeweils einen Halbleiterchip umfassen, der jeweils über die erste und die zweite Leiterbahn mit dem ersten und dem zweiten Richtmaß (4, 5) verbunden ist.

5. Messsystem nach einem der Ansprüche 1 bis 4, wobei das erste Richtmaß (4) und das zweite Richtmaß (5) an einer von den beiden Seitenwänden (1) und dem Querträger (2) befestigt sind.

6. Messsystem (10) nach einem der Ansprüche 1 bis 5, das ferner einen Anwesenheitssensor (13) umfasst, der ausgelegt ist, um eine Anwesenheit eines Individuums in dem Durchgangskanal (9) zu bestimmen, wobei die Verarbeitungseinheit (15) ausgelegt ist, um das elektronische Bild nur dann zu erzeugen, wenn der Anwesenheitssensor (13) ein Objekt innerhalb des Durchgangskanals (9) erkennt.

7. Messsystem (10) nach Anspruch 2, wobei die Infrarotkamera (12) und die Kamera für sichtbares Spektrum (6) an einem Arm (7) angebracht sind, der am Querträger (2) befestigt ist und sich ab einem Ausgang (9b) des Portals (11) erstreckt.

8. Messsystem (10) nach einem der Ansprüche 1 bis 7, das ferner ein vorzugsweise blinkendes Licht (30) umfasst, das in der Nähe der Infrarotkamera (12) befestigt ist, um einen Blick eines Individuums anzuziehen, das den Durchgangskanal (9) durchquert.

9. Messsystem (10) nach einem der Ansprüche 1 bis 8, wobei das Sichtfeld (32) der Infrarotkamera (12) einen vertikalen Winkel ($\varphi$) und einen horizontalen Winkel ($\theta$) größer oder gleich 30° und kleiner oder gleich 120° aufweist.

10. Verfahren zum Messen einer Körpertemperatur eines Individuums mithilfe eines Messsystems (10) nach einem der Ansprüche 1 bis 9, das die folgenden Schritte umfasst:

    S1: Erstellen eines elektronischen Bildes eines Abschnitts des Durchgangskanals (9), in dem sich ein Individuum befindet, wobei das elektronische Bild eine Vielzahl von Infrarotbildpixeln umfasst, die für einen Temperaturwert repräsentativ sind, der von einem entsprechenden Infrarotpixel einer Infrarotpixelmatrix einer Infrarotkamera (12) empfangen wurde, wobei ein erstes Richtmaß (4) und ein zweites Richtmaß (5) im Sichtfeld (32) der Infrarotkamera (12) derart positioniert ist, dass das elektronische Bild Infrarotbildpixel umfasst, die für den Temperaturwert des ersten Richtmaßes (4) und des zweiten Richtmaßes (5) repräsentativ sind;
    S4: Identifizieren der Infrarotbildpixel, die mindestens einem Teil eines Gesichts des Individuums entsprechen, vorzugsweise mindestens einem inneren Augenwinkel;
    S5: Bestimmen einer momentanen Temperatur einer Leiterbahn einer ersten Wärmesonde (20), die in der Mitte einer Heizfläche des ersten Richtmaßes (4) befestigt ist, und einer Leiterbahn einer zweiten Wärmesonde (22), die in der Mitte einer Heizfläche des zweiten Richtmaßes (5) befestigt ist;
    S6: Bestimmen des Temperaturwerts der Bildpixel, die dem ersten Richtmaß (4) und dem zweiten Richtmaß (5) entsprechen, in dem elektronischen Bild;
    S7: Ableiten daraus eines Verstärkungskoeffizienten ($k_1$) und eines Verschiebungskoeffizienten ($k_2$) der Infrarotkamera (12); und
    S8: Anwenden des Verstärkungskoeffizienten ($k_1$) und des Verschiebungskoeffizienten ($k_2$) auf den Temperaturwert, der jedem Bildpixel zugeordnet ist, um ein korrigiertes elektronisches Bild zu erhalten; und
    S9: Bestimmen eines maximalen Temperaturwerts, der den identifizierten Infrarotbildpixeln zugeordnet ist, und Ableiten einer Körpertemperatur der Person daraus.

11. Messverfahren nach Anspruch 10, das vor dem Schritt S9 ferner folgende Schritte umfasst:

    S2: Erstellen eines sichtbaren Bildes des gesamten oder eines Teils des Abschnitts des Durchgangskanals (9), wobei das sichtbare Bild eine Vielzahl sichtbarer Bildpixel umfasst;

S3: Identifizieren der sichtbaren Bildpixel, die mindestens einem Teil eines Gesichts des Individuums entsprechen, vorzugsweise mindestens eines inneren Augenwinkels; und derart, dass Schritt S4 das Abgleichen des sichtbaren Bildes und des elektronischen Bildes umfasst, um die Infrarotbildpixel zu identifizieren, die den in Schritt S3 identifizierten sichtbaren Bildpixeln entsprechen,

wobei die Schritte S1 und S2 gleichzeitig erfolgen können.

**12.** Verfahren nach einem der Ansprüche 10 oder 11, das die Anwendung eines vorbestimmter Kompensationskoeffizienten ($k_3$) auf den Temperaturwert jedes Bildpixels oder auf den in Schritt S9 bestimmten maximalen Temperaturwert ($T_{max}$) umfasst.

**13.** Verfahren nach einem der Ansprüche 10 bis 12, das ferner vor Schritt S1 einen Schritt S0 des Bestimmens einer Anwesenheit eines Individuums im Portal (11) umfasst, wobei die Schritte S1 bis S9 nur dann durchgeführt werden, wenn ein Individuum im Durchgangskanal (9) anwesend ist.

**Claims**

**1.** Measurement system (10) of an individual's body temperature comprising:

an archway (11) comprising two side panels (1) connected by a cross member (2) and together delimiting a passageway (9) for an individual;
an infrared camera (12) comprising an infrared detection chip (25) comprising an infrared pixel matrix and being configured to convert an infrared radiation received by each infrared pixel into a corresponding temperature value and to generate an electronic image comprising a plurality of infrared image pixels, each infrared image pixel being representative of the temperature value received by a corresponding infrared pixel, the infrared camera (12) being fixed onto the archway (11) so that a field of view (32) of the infrared camera (12) covers all or part of the portion of the passageway (9);
a processing unit (15) configured to identify the infrared pixels corresponding to at least a part of an individual's face and to determine a maximum temperature value associated with the identified infrared pixels and to deduce therefrom a body temperature of the individual; and
a calibration module (3) comprising a first reference target (4) and a second reference target (5) positioned in the field of view (32) of the infrared camera (12) so that the electronic image comprises the infrared image pixels representative of the temperature value of the first reference target (4) and the second reference target (5);
the calibration module further comprises a first thermal sensor (20) fixed at the center of a heated surface of the first reference target (4) and configured to measure an instantaneous temperature of the first reference target (4) and a second thermal sensor (22) fixed at the center of a heated surface of the second reference target (5) and configured to measure an instantaneous temperature of the second reference target (5), the first and the second thermal sensors (20, 22) being connected to the first and second reference targets (4, 5), respectively, by means of the first and a second conductive track,
the processing unit being further configured to :

determine an instantaneous temperature of the first and the second conductive tracks and deduce therefrom the instantaneous temperature of the first and second reference targets (4, 5) respectively; and
determine a gain coefficient ($k_1$) and an offset coefficient ($k_2$) from the respective instantaneous temperatures of the first reference target (4) and the second reference target (5) and temperature values of the first reference target (4) and the second reference target (5) in the electronic image and to apply the gain coefficient ($k_1$) and the offset coefficient ($k_2$) thus determined to the temperature value associated with each infrared image pixel so as to obtain a corrected electronic image, the processing unit (15) being configured to determine the maximum temperature value from the corrected electronic image.

**2.** Measurement system (10) according to claim 1, further comprising a visible spectrum camera (6) comprising a visible spectrum detection chip (29) comprising a visible pixel matrix and being configured to generate a visible image comprising a plurality of visible image pixels, the visible spectrum camera (6) being fixed onto the archway (11) so that a field of view (31) of the visible spectrum camera (6) covers at least a portion of the passageway (9), the processing unit being configured to match the visible image and the electronic image so as to identify the infrared image pixels corresponding to the visible image pixels identified.

**3.** Measurement system (10) according to one of claims 1 to 2, wherein the processing unit (15) is also configured to apply a predetermined compensation coefficient ($k_3$) to the temperature value of each infrared image pixel.

4. Measurement system (10) according to one of claims 1 to 3, wherein the first and second thermal sensors (20, 22) each comprise a semiconductor chip connected to the first and second reference targets (4, 5), respectively, by means of the first and second conductive tracks.

5. Measurement system according to one of claims 1 to 4, wherein the first reference target (4) and the second reference target (5) are fixed onto one of the two side panels (1) and the cross member (2).

6. Measurement system (10) according to one of claims 1 to 5, also comprising a presence sensor (13) configured to determine a presence of an individual in the passageway (9), the processing unit (15) being configured to generate the electronic image only when the presence sensor (13) detects an object inside the passageway (9).

7. Measurement system (10) according to claim 2, wherein the infrared camera (12) and the visible spectrum camera (6) are mounted on an arm (7) fixed onto the cross member (2) and extending from an exit (9b) of the archway (11).

8. Measurement system (10) according to one of claims 1 to 7, also comprising a light (30), preferably flashing, fixed near the infrared camera (12) so as to draw the gaze of an individual passing through the passageway (9).

9. Measurement system (10) according to one of claims 1 to 8, wherein the field of view (32) of the infrared camera (12) has a vertical angle ($\varphi$) and a horizontal angle ($\theta$) greater than or equal to 30° and less than or equal to 120°.

10. Measurement method for an individual's body temperature using a measurement system (10) according to one of claims 1 to 9, comprising the following steps:

S1: producing an electronic image of a portion of the passageway (9) in which an individual is located, the electronic image comprising a plurality of infrared image pixels representative of a temperature value received by a corresponding infrared pixel of a matrix of infrared pixels of an infrared camera (12), a first reference target (4) and a second reference target (5) being positioned in the field of view (32) of the infrared camera (12) so that the electronic image comprises infrared image pixels representative of the temperature value of the first reference target (4) and the second reference target (5);
S4: identifying the infrared image pixels corresponding to at least part of an individual's face, preferably at least one inner corner of the eyes;
S5: determining an instantaneous temperature of a conductive track fixed at the center of a heated surface of the first reference target (4) and of a conductive track fixed at the center of a heated surface of the second reference target (5);
S6: determining in the electronic image the temperature value of the image pixels corresponding to the first reference target (4) and to the second reference target (5);
S7: deducing therefrom a gain coefficient ($k_1$) and an offset coefficient ($k_2$) for the infrared camera (12); and
S8: applying the gain coefficient ($k_1$) and the offset coefficient ($k_2$) to the temperature value associated with each image pixel so as to obtain a corrected electronic image; and
S9: determining a maximum temperature value associated with the identified infrared image pixels and deducing therefrom a body temperature of the individual.

11. Measurement method according to claim 10, also comprising the following steps, prior to step S9:

S2: producing a visible image of all or part of the portion of the passageway (9), the visible image comprising a plurality of visible image pixels;
S3: identifying the visible image pixels corresponding to at least a part of an individual's face, preferably at least one inner corner of the eyes; and

such that step S4 comprises matching the visible image and the electronic image so as to identify the infrared image pixels corresponding to the visible image pixels identified in step S3; steps S1 and S2 being optionally simultaneous.

12. Method according to one of claims 10 or 11, comprising applying a predetermined compensation coefficient ($k_3$) to the temperature value of each image pixel or to the maximum temperature value (Tmax) determined in step S9.

13. Method according to one of claims 10 to 12, further comprising, prior to step S1, a step S0 of determination of a presence of an individual in the archway (11), steps S1 to S9 being implemented only when an individual is present in the passageway (9).

**Fig. 1**

**Fig. 2**

Fig. 3

EP 3 929 549 B1

Fig. 4

**Fig. 5**

Fig. 6

Fig. 7

Fig. 8

Début

Mesurer une température instantanée
du premier étalon

Ajuster une température du premier étalon à
la première température de référence

Mesurer une température instantanée
du deuxième étalon

Ajuster une température du deuxième étalon à
la deuxième température de référence

Non ← Individu détecté? ⟋S0

Oui

Générer une image électronique
de l'individu — S1

Déterminer une température instantanée
du premier et du deuxième étalon — S5

Déterminer la valeur de température des pixels
image correspondant au premier et au deuxième étalon — S6

**Fig. 9**

En déduire le coefficient de gain et
le coefficient de décalage de la caméra infrarouge — S7

Appliquer les coefficient de gain et de
décalage aux valeurs de température — S8

Appliquer un coefficient de compensation
aux valeurs de température

Déterminer la valeur de température maximale $T_{max}$ — S9

Signal optique vert et confirmation
acoustique d'analyse validée ← Non — $T_{max} > T_{seuil}$ ?

Oui

Signal optique rouge et alarme acoustique

Réaliser une image électronique du canal de passage —S1

Réaliser une image visible du canal de passage —S2

Identifier les pixels image visibles correspondant à un visage de l'individu, de préférence au moins un coin interne des yeux —S3

Mettre en correspondance l'image visible et l'image électronique de sorte à identifier les pixels images infrarouges correspondant aux pixels images visibles identifiés à l'étape S3 —S4

Déterminer la valeur de température maximale $T_{max}$ —S9

**Fig. 10**

**Fig. 11**

EP 3 929 549 B1

## RÉFÉRENCES CITÉES DANS LA DESCRIPTION

**Documents brevets cités dans la description**

- US 2007153871 A1
- WO 2004097389 A2